# EUROPEAN PATENT APPLICATION

(11) **EP 0 753 528 A1**
(43) Date of publication of application: **15.01.1997**
(21) Application number: 95907861.9
(22) Date of filing: 08.02.1995
(51) Int. Cl.: C07K 14/72, C12N 15/00, C12N 5/00, C12P 21/02, C07K 16/28, C12P 21/08, A61K 38/17, G01N 33/566, A61K 39/395

(54) **PROSTAGLANDIN I 2? RECEPTOR**

(30) Priority: 10.02.1994 JP 37703/94
(71) Applicant: ONO PHARMACEUTICAL CO., LTD., Osaka-shi Osaka 541 (JP)
(72) Inventor: ICHIKAWA, Atsushi, Osaka 569 (JP); NAKAO, Kazuwa, Kyoto-shi Kyoto 610-11 (JP); NARUMIYA, Shuh, Kyoto 610-11 (JP)
(74) Representative: Bentham, Stephen
(86) International application number: JP9500175
(87) International publication number: WO9521863

(57) **Abstract**

A human and rat PGI₂ receptor; a process for producing the same; a DNA coding for a peptide of the same; a fragment which selectively hybridizes with the sequence of the DNA; a replication or expression vector comprising the DNA; a host cell transformed by the vector; a monoclonal or polyclonal antibody of the polypeptide; a pharmaceutical composition containing the polypeptide or the antibody; and a method of screening a compound having a PGI₂ agonist or antagonist activity by using the polypeptide or the host cell. As the above polypeptide itself specifically binds to PIG₂, it can be used as a preventive or remedy for diseases caused by PGI₂ overproduction, such as hemorrhagic diseases. Also it can be used for screening a substance having a PGI₂ agonist or antagonist activity.

Sequence Listing

(1) GENERAL INFORMATION:

.........

(2) INFORMATION FOR SEQ ID NO: 1:
(i) SEQUENCE CHARACTERISTICS:
(A) LENGTH 386 amino acids
(B) TYPE: amino acid
(D) TOPOLOGY: linear
(ii) MOLECULE TYPE: protein
(xi) SEQUENCE DESCRIPTION: SEQ ID NO: 1


1               5                   10                  15

            20                  25                  30

        35                  40                  45

    50                  55                  60

65                  70                  75                  80

                85                  90                  95

            100                 105                 110

        115                 120                 125

    130                 135                 140

145                 150                 155                 160

                165                 170                 175

            180                 185                 190

        195                 200                 205

    210                 215                 220

225                 230                 235                 240


                245                 250                 255

            260                 265                 270

        275                 280                 285

    290                 295                 300

305                 310                 315                 320

                325                 330                 335

            340                 345                 350

        355                 360                 365

    370                 375                 380

385

(2) INFORMATION FOR SEQ ID NO: 2:
    (i) SEQUENCE CHARACTERISTICS:
        (A) LENGTH 1158 base pairs
        (B) TYPE: nucleic acid
        (C) STRANDEDNESS: single
        (D) TOPOLOGY: linear
    (ii) MOLECULE TYPE: cDNA to mRNA
    (xi) SEQUENCE DESCRIPTION: SEQ ID NO: 2

ATGGCGGATT CGTGCAGGAA CCTCACCTAC GTGCGGGGCT CGGTGGGGCC GGCCACCAGC   60
ACCCTGATGT TCGTGGCCGG TGTGGTGGGC AACGGGCTGG CCCTGGGCAT CCTGAGCGCA  120
CGGCGACCGG CGCGCCCCTC GGCCTTCGCG GTGCTGGTGA CCGGACTGGC GGCCACCGAC  180
CTGCTGGGCA CCAGCTTCCT GAGCCCGGCC GTGTTCGTGG CCTATGCGCG CAACAGCTCC  240
CTGCTGGGCC TGGCCCGAGG CGGCCCCGCC CTGTGCGATG CCTTCGCCTT CGCCATGACC  300
TTCTTCGGCC TGGCGTCCAT GCTCATCCTC TTTGCCATGG CCGTGGAGCG CTGCCTGGCG  360
CTGAGCCACC CCTACCTCTA CGCGCAGCTG GACGGGCCCC GCTGCGCCCG CCTGGCGCTG  420
CCAGCCATCT ACGCCTTCTG CGTCCTCTTC TGCGCGCTGC CCCTGCTGGG CCTGGGCCAA  480
CACCAGCAGT ACTGCCCCGG CAGCTGGTGC TTCCTCCGCA TGCGCTGGGC CCAGCCGGGC  540
GGCGCCGCCT TCTCGCTGGC CTACGCCGGC CTGGTGGCCC TGCTGGTGGC TGCCATCTTC  600
CTCTGCAACG GCTCGGTCAC CCTCAGCCTC TGCCGCATGT ACCGCCAGCA GAAGCGCCAC  660

CAGGGCTCTC TGGGTCCACG GCCGCGCACC GGAGAGGACG AGGTGGACCA CCTGATCCTG 720
CTGGCCCTCA TGACAGTGGT CATGGCCGTG TGCTCCCTGC CTCTCACGAT CCGCTGCTTC 780
ACCCAGGCTG TCGCCCCTGA CAGCAGCAGT GAGATGGGGG ACCTCCTTGC CTTCCGCTTC 840
TACGCCTTCA ACCCCATCCT GGACCCCTGG GTCTTCATCC TTTTCCGCAA GGCTGTCTTC 900
CAGCGACTCA AGCTCTGGGT CTGCTGCCTG TGCCTCGGGC CTGCCCACGG AGACTCGCAG 960
ACACCCCTTT CCCAGCTCGC CTCAGGGAGG AGGGACCCAA GGGCCCCCTC TGCTCCTGTG 1020
GGAAAGGAGG GGAGCTGCGT GCCTTTGTCG GCTTGGGGCG AGGGGCAGGT GGAGCCCTTG 1080
CCTCCCACAC AGCAGTCCAG CGGCAGCGCC GTGGGAACGT CGTCCAAAGC AGAAGCCAGC 1140
GTCGCCTGCT CCCTCTGC 1158

(2) INFORMATION FOR SEQ ID NO: 3:
(i) SEQUENCE CHARACTERISTICS:
(A) LENGTH 1979 base pairs
(B) TYPE: nucleic acid
(C) STRANDEDNESS: single
(D) TOPOLOGY: linear
(ii) MOLECULE TYPE: cDNA to mRNA
(xi) SEQUENCE DESCRIPTION: SEQ ID NO: 3

AGGCAGCGAG AGACACGAGG AGCAAAGCAA GTGAAGGCAC AGACGCACGG GACAGGAGAG 60
CCTGGGCAAG ACTGGAGAGC CCAGACCTGG GATGGCGGAT TCGTGCAGGA ACCTCACCTA 120
CGTGCGGGGC TCGGTGGGGC CGGCCACCAG CACCCTGATG TTCGTGGCCG GTGTGGTGGG 180
CAACGGGCTG GCCCTGGGCA TCCTGAGCGC ACGGCGACCG GCGCGCCCCT CGGCCTTCGC 240
GGTGCTGGTG ACCGGACTGG CGGCCACCGA CCTGCTGGGC ACCAGCTTCC TGAGCCCGGC 300
CGTGTTCGTG GCCTATGCGC GCAACAGCTC CCTGCTGGGC CTGGCCCGAG GCGGCCCCGC 360
CCTGTGCGAT GCCTTCGCCT TCGCCATGAC CTTCTTCGGC CTGGCGTCCA TGCTCATCCT 420
CTTTGCCATG GCCGTGGAGC GCTGCCTGGC GCTGAGCCAC CCCTACCTCT ACGCGCAGCT 480
GGACGGGCCC CGCTGCGCCC GCCTGGCGCT GCCAGCCATC TACGCCTTCT GCGTCCTCTT 540
CTGCGCGCTG CCCCTGCTGG GCCTGGGCCA ACACCAGCAG TACTGCCCCG GCAGCTGGTG 600
CTTCCTCCGC ATGCGCTGGG CCCAGCCGGG CGGCGCCGCC TTCTCGCTGG CCTACGCCGG 660
CCTGGTGGCC CTGCTGGTGG CTGCCATCTT CCTCTGCAAC GGCTCGGTCA CCCTCAGCCT 720
CTGCCGCATG TACCGCCAGC AGAAGCGCCA CCAGGGCTCT CTGGGTCCAC GGCCGCGCAC 780
CGGAGAGGAC GAGGTGGACC ACCTGATCCT GCTGGCCCTC ATGACAGTGG TCATGGCCGT 840
GTGCTCCCTG CCTCTCACGA TCCGCTGCTT CACCCAGGCT GTCGCCCCTG ACAGCAGCAG 900
TGAGATGGGG GACCTCCTTG CCTTCCGCTT CTACGCCTTC AACCCCATCC TGGACCCCTG 960
GGTCTTCATC CTTTTCCGCA AGGCTGTCTT CCAGCGACTC AAGCTCTGGG TCTGCTGCCT 1020
GTGCCTCGGG CCTGCCCACG GAGACTCGCA GACACCCCTT TCCCAGCTCG CCTCAGGGAG 1080
GAGGGACCCA AGGGCCCCCT CTGCTCCTGT GGGAAAGGAG GGGAGCTGCG TGCCTTTGTC 1140
GGCTTGGGGC GAGGGGCAGG TGGAGCCCTT GCCTCCCACA CAGCAGTCCA GCGGCAGCGC 1200
CGTGGGAACG TCGTCCAAAG CAGAAGCCAG CGTCGCCTGC TCCCTCTGCT GACATTTCAA 1260

GCTGACCCTG TGATCTCTGC CCTGTCTTCG GGCGACAGGA GCCAGAAAAT CAGGGACATG 1320
GCTGATGGCT GCGGATGCTG GAACCTTGGC CCCCAAACTC TGGGGCCGAT CAGCTGCTGT 1380
TTCTCCTGCG GCAGGGCAGT CGCTGCTGGC TCTGGGAAGA GAGTGAGGGA CAGAGGAAAC 1440
GTTTATCCTG GAGTGCAGAA AGAATGGTTC TCTCAAAATA ACCAGTGGCC TGGCCGACCT 1500
GCTCTGGCCC TGGATTCCCC ATCCATCTCA TTGTCTAAAT ATTTAGAAGG CGGAGAAGTT 1560
CCCAGAGGCT TCTGTACAGT CAGGTCTGCT CTGGTCTGGG TGCTGGCTCC AATCTGCGTC 1620
CACTTAGGAG GCCCAACTGC CCACCCCAAG TCCCCAGGGG ATGGCCCTCC CCCTCTACCA 1680
AGCCACTCCA AGAGCCAGCC CCTTTCTGCT CCACAAAAAC CACAGTTATT GGAAAAGCTC 1740
CCTGCCTTCC CTTGCCGCTG GTCCCCCACC AGGCTTGGGA GCCCTGGCAT CCCAAAGGGG 1800
CAACGGGAGG AAGGGGAGGC TGCTGCATTG TGGGTGATGA CGTAGGACAT GTGCTTGGTA 1860
CAAAAAGGGC CTGAGACATT CCACCTAGCT TGACTGGCTG CAAGATGAGA ACTGGGGGGG 1920
TGCAGGTGGT GGGGAGACAG ATGGAGAAGC TGGCAGATGA AGGGTGGGGG CTGCGGATC 1979

(2) INFORMATION FOR SEQ ID NO: 4:
(i) SEQUENCE CHARACTERISTICS:
(A) LENGTH 1979 base pairs
(B) TYPE: nucleic acid
(C) STRANDEDNESS: single
(D) TOPOLOGY: linear
(ii) MOLECULE TYPE: cDNA to mRNA
(vi) ORIGINAL SOURCE
(A) ORGANISM: Homo sapiens
(G) CELL TYPE: megakaryocyte leukemia cell
(H) CELL LINE: CMK
(ix) FEATURE
(A) NAME/KEY: CDS
(B) LOCATION: 92 to 1249
(C) IDENTIFICATION METHOD: P
(xi) SEQUENCE DESCRIPTION: SEQ ID NO: 4

AGGCAGCGAG AGACACGAGG AGCAAAGCAA GTGAAGGCAC AGACGCACGG GACAGGAGAG 60
CCTGGGCAAG ACTGGAGAGC CCAGACCTGG G ATG GCG GAT TCG TGC AGG AAC 112

1               5
CTC ACC TAC GTG CGG GGC TCG GTG GGG CCG GCC ACC AGC ACC CTG ATG 160

        10                  15                  20
TTC GTG GCC GGT GTG GTG GGC AAC GGG CTG GCC CTG GGC ATC CTG AGC 208

    25                  30                  35
GCA CGG CGA CCG GCG CGC CCC TCG GCC TTC GCG GTG CTG GTG ACC GGA 256


40                  45                  50                  55
CTG GCG GCC ACC GAC CTG CTG GGC ACC AGC TTC CTG AGC CCG GCC GTG     304

                60                  65                  70
TTC GTG GCC TAT GCG CGC AAC AGC TCC CTG CTG GGC CTG GCC CGA GGC     352

            75                  80                  85
GGC CCC GCC CTG TGC GAT GCC TTC GCC TTC GCC ATG ACC TTC TTC GGC     400

        90                  95                  100
CTG GCG TCC ATG CTC ATC CTC TTT GCC ATG GCC GTG GAG CGC TGC CTG     448

    105                 110                 115
GCG CTG AGC CAC CCC TAC CTC TAC GCG CAG CTG GAC GGG CCC CGC TGC     496

120                 125                 130                 135
GCC CGC CTG GCG CTG CCA GCC ATC TAC GCC TTC TGC GTC CTC TTC TGC     544

                140                 145                 150
GCG CTG CCC CTG CTG GGC CTG GGC CAA CAC CAG CAG TAC TGC CCC GGC     592

            155                 160                 165
AGC TGG TGC TTC CTC CGC ATG CGC TGG GCC CAG CCG GGC GGC GCC GCC     640

        170                 175                 180
TTC TCG CTG GCC TAC GCC GGC CTG GTG GCC CTG CTG GTG GCT GCC ATC     688

    185                 190                 195
TTC CTC TGC AAC GGC TCG GTC ACC CTC AGC CTC TGC CGC ATG TAC CGC     736

200                 205                 210                 215
CAG CAG AAG CGC CAC CAG GGC TCT CTG GGT CCA CGG CCG CGC ACC GGA     784

                220                 225                 230
GAG GAC GAG GTG GAC CAC CTG ATC CTG CTG GCC CTC ATG ACA GTG GTC     832

            235                 240                 245
ATG GCC GTG TGC TCC CTG CCT CTC ACG ATC CGC TGC TTC ACC CAG GCT     880

        250                 255                 260
GTC GCC CCT GAC AGC AGC AGT GAG ATG GGG GAC CTC CTT GCC TTC CGC     928

    265                 270                 275
TTC TAC GCC TTC AAC CCC ATC CTG GAC CCC TGG GTC TTC ATC CTT TTC     976


280                 285                 290                 295
CGC AAG GCT GTC TTC CAG CGA CTC AAG CTC TGG GTC TGC TGC CTG TGC   1024

            300                 305                 310
CTC GGG CCT GCC CAC GGA GAC TCG CAG ACA CCC CTT TCC CAG CTC GCC   1072

        315                 320                 325
TCA GGG AGG AGG GAC CCA AGG GCC CCC TCT GCT CCT GTG GGA AAG GAG   1120

    330                 335                 340
GGG AGC TGC GTG CCT TTG TCG GCT TGG GGC GAG GGG CAG GTG GAG CCC   1168

    345                 350                 355
TTG CCT CCC ACA CAG CAG TCC AGC GGC AGC GCC GTG GGA ACG TCG TCC   1216

360                 365                 370                 375
AAA GCA GAA GCC AGC GTC GCC TGC TCC CTC TGC TGACATTTCA AGCTGACCCT 1269

            380                 385
GTGATCTCTG CCCTGTCTTC GGGCGACAGG AGCCAGAAAA TCAGGGACAT GGCTGATGGC 1329
TGCGGATGCT GGAACCTTGG CCCCCAAACT CTGGGGCCGA TCAGCTGCTG TTTCTCCTGC 1389
GGCAGGGCAG TCGCTGCTGG CTCTGGGAAG AGAGTGAGGG ACAGAGGAAA CGTTTATCCT 1449
GGAGTGCAGA AAGAATGGTT CTCTCAAAAT AACCAGTGGC CTGGCCGACC TGCTCTGGCC 1509
CTGGATTCCC CATCCATCTC ATTGTCTAAA TATTTAGAAG GCGGAGAAGT TCCCAGAGGC 1569
TTCTGTACAG TCAGGTCTGC TCTGGTCTGG GTGCTGGCTC CAATCTGCGT CCACTTAGGA 1629
GGCCCAACTG CCCACCCCAA GTCCCCAGGG GATGGCCCTC CCCCTCTACC AAGCCACTCC 1689
AAGAGCCAGC CCCTTTCTGC TCCACAAAAA CCACAGTTAT TGGAAAAGCT CCCTGCCTTC 1749
CCTTGCCGCT GGTCCCCCAC CAGGCTTGGG AGCCCTGGCA TCCCAAAGGG GCAACGGGAG 1809
GAAGGGGAGG CTGCTGCATT GTGGGTGATG ACGTAGGACA TGTGCTTGGT ACAAAAAGGG 1869
CCTGAGACAT TCCACCTAGC TTGACTGGCT GCAAGATGAG AACTGGGGGG GTGCAGGTGG 1929
TGGGGAGACA GATGGAGAAG CTGGCAGATG AAGGGTGGGG GCTGCGGATC            1979

(2) INFORMATION FOR SEQ ID NO: 5:
    (i) SEQUENCE CHARACTERISTICS:
        (A) LENGTH 1158 base pairs
        (B) TYPE: nucleic acid
        (C) STRANDEDNESS: single
        (D) TOPOLOGY: linear
    (ii) MOLECULE TYPE: cDNA to mRNA
    (xi) SEQUENCE DESCRIPTION: SEQ ID NO: 5

ATGGCGGATT CGTGCAGGAA CCTCACCTAC GTGCGGGGCT CGGTGGGGCC GGCCACCAGC 60
ACCCTGATGT TCGTGGCCGG TGTGGTGGGC AACGGGCTGG CCCTGGGCAT CCTGAGCGCA 120
CGGCGACCGG CGCGCCCCTC GGCCTTCGCG GTGCTGGTCA CCGGACTGGC GGCCACCGAC 180
CTGCTGGGCA CCAGCTTCCT GAGCCCGGCC GTGTTCGTGG CCTATGCGCG CAACAGCTCC 240
CTGCTGGGCC TGGCCCGAGG CGGCCCCGCC CTGTGCGATG CCTTCGCCTT CGCCATGACC 300
TTCTTCGGCC TGGCGTCCAT GCTCATCCTC TTTGCCATGG CCGTGGAGCG CTGCCTGGCG 360
CTGAGCCACC CCTACCTCTA CGCGCAGCTG GACGGGCCCC GCTGCGCCCG CCTGGCGCTG 420
CCAGCCATCT ACGCCTTCTG CGTCCTCTTC TGCGCGCTGC CCCTGCTGGG CCTGGGCCAA 480
CACCAGCAGT ACTGCCCCGG CAGCTGGTGC TTCCTCCGCA TGCGCTGGGC CCAGCCGGGC 540
GGCGCCGCCT TCTCGCTGGC CTACGCCGGC CTGGTGGCCC TGCTGGTGGC TGCCATCTTC 600
CTCTGCAACG GCTCGGTCAC CCTCAGCCTC TGCCGCATGT ACCGGCAGCA GAAGCGCCAC 660
CAGGGCTCTC TGGGTCCACG GCCGCGCACC GGAGAGGACG AGGTGGACCA CCTGATCCTG 720
CTGGCCCTCA TGACAGTGGT CATGGCCGTG TGCTCCCTGC CTCTCACGAT CCGCTGCTTC 780
ACCCAGGCTG TCGCCCCTGA CAGCAGCAGT GAGATGGGGG ACCTCCTTGC CTTCCGCTTC 840
TACGCCTTCA ACCCCATCCT GGACCCCTGG GTCTTCATCC TTTTCCGCAA GGCTGTCTTC 900
CAGCGACTCA AGCTCTGGGT CTGCTGCCTG TGCCTCGGGC CTGCCCACGG AGACTCGCAG 960
ACACCCCTTT CCCAGCTCGC CTCCGGGAGG AGGGACCCAA GGGCCCCCTC TGCTCCTGTG 1020
GGAAAGGAGG GGAGCTGCGT GCCTTTGTCG GCTTGGGGCG AGGGGCAGGT GGAGCCCTTG 1080
CCTCCCACAC AGCAGTCCAG CGGCAGCGCC GTGGGAACGT CGTCCAAAGC AGAAGCCAGC 1140
GTCGCCTGCT CCCTCTGC 1158

(2) INFORMATION FOR SEQ ID NO: 6:
(i) SEQUENCE CHARACTERISTICS:
(A) LENGTH 1320 base pairs
(B) TYPE: nucleic acid
(C) STRANDEDNESS: single
(D) TOPOLOGY: linear
(ii) MOLECULE TYPE: cDNA to mRNA
(xi) SEQUENCE DESCRIPTION: SEQ ID NO: 6

AGCAAGTGAA GGCACAGACG CACGGGACAG GAGAGCCTGG GCAAGACTGG AGAGCCCAGA 60
CCTGGGATGG CGGATTCGTG CAGGAACCTC ACCTACGTGC GGGGCTCGGT GGGGCCGGCC 120
ACCAGCACCC TGATGTTCGT GGCCGGTGTG GTGGGCAACG GGCTGGCCCT GGGCATCCTG 180
AGCGCACGGC GACCGGCGCG CCCCTCGGCC TTCGCGGTGC TGGTCACCGG ACTGGCGGCC 240
ACCGACCTGC TGGGCACCAG CTTCCTGAGC CCGGCCGTGT TCGTGGCCTA TGCGCGCAAC 300
AGCTCCCTGC TGGGCCTGGC CCGAGGCGGC CCCGCCCTGT GCGATGCCTT CGCCTTCGCC 360
ATGACCTTCT TCGGCCTGGC GTCCATGCTC ATCCTCTTTG CCATGGCCGT GGAGCGCTGC 420
CTGGCGCTGA GCCACCCCTA CCTCTACGCG CAGCTGGACG GGCCCCGCTG CGCCCGCCTG 480

GCGCTGCCAG CCATCTACGC CTTCTGCGTC CTCTTCTGCG CGCTGCCCCT GCTGGGCCTG 540
GGCCAACACC AGCAGTACTG CCCCGGCAGC TGGTGCTTCC TCCGCATGCG CTGGGCCCAG 600
CCGGGCGGCG CCGCCTTCTC GCTGGCCTAC GCCGGCCTGG TGGCCCTGCT GGTGGCTGCC 660
ATCTTCCTCT GCAACGGCTC GGTCACCCTC AGCCTCTGCC GCATGTACCG CCAGCAGAAG 720
CGCCACCAGG GCTCTCTGGG TCCACGGCCG CGCACCGGAG AGGACGAGGT GGACCACCTG 780
ATCCTGCTGG CCCTCATGAC AGTGGTCATG GCCGTGTGCT CCCTGCCTCT CACGATCCGC 840
TGCTTCACCC AGGCTGTCGC CCCTGACAGC AGCAGTGAGA TGGGGGACCT CCTTGCCTTC 900
CGCTTCTACG CCTTCAACCC CATCCTGGAC CCCTGGGTCT TCATCCTTTT CCGCAAGGCT 960
GTCTTCCAGC GACTCAAGCT CTGGGTCTGC TGCCTGTGCC TCGGGCCTGC CCACGGAGAC 1020
TCGCAGACAC CCCTTTCCCA GCTCGCCTCC GGGAGGAGGG ACCCAAGGGC CCCCTCTGCT 1080
CCTGTGGGAA AGGAGGGGAG CTGCGTGCCT TTGTCGGCTT GGGGCGAGGG GCAGGTGGAG 1140
CCCTTGCCTC CCACACAGCA GTCCAGCGGC AGCGCCGTGG GAACGTCGTC CAAAGCAGAA 1200
GCCAGCGTCG CCTGCTCCCT CTGCTGACAT TTCAAGCTGA CCCTGTGATC TCTGCCCTGT 1260
CTTCGGGCGA CAGGAGCCAG AAAATCAGGG ACATGGCTGA TGGCTGCGGA TGCTGGAACC 1320

(2) INFORMATION FOR SEQ ID NO: 7:
(i) SEQUENCE CHARACTERISTICS:
(A) LENGTH 1320 base pairs
(B) TYPE: nucleic acid
(C) STRANDEDNESS: single
(D) TOPOLOGY: linear
(ii) MOLECULE TYPE: cDNA to mRNA
(vi) ORIGINAL SOURCE
(A) ORGANISM: Homo sapiens
(F) TISSUE TYPE: lung
(ix) FEATURE
(A) NAME/KEY: CDS
(B) LOCATION: 67 to 1224
(C) IDENTIFICATION METHOD: P
(xi) SEQUENCE DESCRIPTION: SEQ ID NO: 7

AGCAAGTGAA GGCACAGACG CACGGGACAG GAGAGCCTGG GCAAGACTGG AGAGCCCAGA 60
CCTGGG ATG GCG GAT TCG TGC AGG AAC CTC ACC TAC GTG CGG GGC TCG 108

1               5                   10
GTG GGG CCG GCC ACC AGC ACC CTG ATG TTC GTG GCC GGT GTG GTG GGC 156

15                  20                  25                  30
AAC GGG CTG GCC CTG GGC ATC CTG AGC GCA CGG CGA CCG GCG CGC CCC 204


                35                  40                  45
TCG GCC TTC GCG GTG CTG GTC ACC GGA CTG GCG GCC ACC GAC CTG CTG 252

            50                  55                  60
GGC ACC AGC TTC CTG AGC CCG GCC GTG TTC GTG GCC TAT GCG CGC AAC 300

        65                  70                  75
AGC TCC CTG CTG GGC CTG GCC CGA GGC GGC CCC GCC CTG TGC GAT GCC 348

    80                  85                  90
TTC GCC TTC GCC ATG ACC TTC TTC GGC CTG GCG TCC ATG CTC ATC CTC 396

95                  100                 105                 110
TTT GCC ATG GCC GTG GAG CGC TGC CTG GCG CTG AGC CAC CCC TAC CTC 444

                115                 120                 125
TAC GCG CAG CTG GAC GGG CCC CGC TGC GCC CGC CTG GCG CTG CCA GCC 492

            130                 135                 140
ATC TAC GCC TTC TGC GTC CTC TTC TGC GCG CTG CCC CTG CTG GGC CTG 540

        145                 150                 155
GGC CAA CAC CAG CAG TAC TGC CCC GGC AGC TGG TGC TTC CTC CGC ATG 588

    160                 165                 170
CGC TGG GCC CAG CCG GGC GGC GCC GCC TTC TCG CTG GCC TAC GCC GGC 636

175                 180                 185                 190
CTG GTG GCC CTG CTG GTG GCT GCC ATC TTC CTC TGC AAC GGC TCG GTC 684

                195                 200                 205
ACC CTC AGC CTC TGC CGC ATG TAC CGC CAG CAG AAG CGC CAC CAG GGC 732

            210                 215                 220
TCT CTG GGT CCA CGG CCG CGC ACC GGA GAG GAC GAG GTG GAC CAC CTG 780

        225                 230                 235
ATC CTG CTG GCC CTC ATG ACA GTG GTC ATG GCC GTG TGC TCC CTG CCT 828

    240                 245                 250
CTC ACG ATC CGC TGC TTC ACC CAG GCT GTC GCC CCT GAC AGC AGC AGT 876

255                 260                 265                 270
GAG ATG GGG GAC CTC CTT GCC TTC CGC TTC TAC GCC TTC AAC CCC ATC 924


                275                 280                 285
CTG GAC CCC TGG GTC TTC ATC CTT TTC CGC AAG GCT GTC TTC CAG CGA 972

            290                 295                 300
CTC AAG CTC TGG GTC TGC TGC CTG TGC CTC GGG CCT GCC CAC GGA GAC 1020

        305                 310                 315
TCG CAG ACA CCC CTT TCC CAG CTC GCC TCC GGG AGG AGG GAC CCA AGG 1068

    320                 325                 330
GCC CCC TCT GCT CCT GTG GGA AAG GAG GGG AGC TGC GTG CCT TTG TCG 1116

335                 340                 345                 350
GCT TGG GGC GAG GGG CAG GTG GAG CCC TTG CCT CCC ACA CAG CAG TCC 1164

                355                 360                 365
AGC GGC AGC GCC GTG GGA ACG TCG TCC AAA GCA GAA GCC AGC GTC GCC 1212

            370                 375                 380
TGC TCC CTC TGC TGACATTTCA AGCTGACCCT GTGATCTCTG CCCTGTCTTC 1264

        385
GGGCGACAGG AGCCAGAAAA TCAGGGACAT GGCTGATGGC TGCGGATGCT GGAACC 1320

(2) INFORMATION FOR SEQ ID NO: 8:
(i) SEQUENCE CHARACTERISTICS:
(A) LENGTH 416 amino acids
(B) TYPE: amino acid
(D) TOPOLOGY: linear
(ii) MOLECULE TYPE: protein
(xi) SEQUENCE DESCRIPTION: SEQ ID NO: 8

Met Val Ala Ser Gly Gly Arg Pro Asp Gly Pro Pro Ser Ile Thr Pro
1               5                   10                  15
Glu Ser Pro Leu Ile Val Gly Gly Arg Glu Trp Gln Gly Met Ala Gly
            20                  25                  30
Ser Cys Trp Asn Ile Thr Tyr Val Gln Asp Ser Val Gly Pro Ala Thr
        35                  40                  45
Ser Thr Leu Met Phe Val Ala Gly Val Val Gly Asn Gly Leu Ala Leu

    50                  55                  60
Gly Ile Leu Gly Ala Arg Arg Arg Ser His Pro Ser Ala Phe Ala Val
65                  70                  75                  80
Leu Val Thr Gly Leu Ala Val Thr Asp Leu Leu Gly Thr Cys Phe Leu
                85                  90                  95
Ser Pro Ala Val Phe Val Ala Tyr Ala Arg Asn Ser Ser Leu Leu Gly
            100                 105                 110
Leu Ala His Gly Gly Thr Met Leu Cys Asp Thr Phe Ala Phe Ala Met
        115                 120                 125
Thr Phe Phe Gly Leu Ala Ser Thr Leu Ile Leu Phe Ala Met Ala Val
    130                 135                 140
Glu Arg Cys Leu Ala Leu Ser His Pro Tyr Leu Tyr Ala Gln Leu Asp
145                 150                 155                 160
Gly Pro Arg Cys Ala Arg Leu Ala Leu Pro Ala Ile Tyr Ala Phe Cys
                165                 170                 175
Cys Leu Phe Cys Ser Leu Pro Leu Leu Gly Leu Gly Glu His Gln Gln
            180                 185                 190
Tyr Cys Pro Gly Ser Trp Cys Phe Ile Arg Met Arg Ser Pro Gln Pro
        195                 200                 205
Gly Gly Cys Ala Phe Ser Leu Ala Tyr Ala Ser Leu Met Ala Leu Leu
    210                 215                 220
Val Thr Ser Ile Phe Phe Cys Asn Gly Ser Val Thr Leu Ser Leu Cys
225                 230                 235                 240
His Met Tyr Arg Gln Gln Arg Arg His His Gly Ser Phe Val Pro Thr
                245                 250                 255
Ser Arg Ala Arg Glu Asp Glu Val Tyr His Leu Ile Leu Leu Ala Leu
            260                 265                 270
Met Thr Gly Ile Met Ala Val Cys Ser Leu Pro Leu Thr Ile Arg Gly
        275                 280                 285
Phe Thr Gln Ala Ile Ala Pro Asp Ser Arg Glu Met Gly Asp Leu His
    290                 295                 300
Ala Phe Arg Phe Asn Ala Phe Asn Pro Ile Leu Asp Pro Trp Val Phe
305                 310                 315                 320
Ile Leu Phe Arg Lys Ala Val Phe Gln Arg Leu Lys Phe Trp Leu Cys
                325                 330                 335
Cys Leu Cys Ala Arg Ser Val His Gly Asp Leu Gln Thr Pro Leu Ser
            340                 345                 350
Arg Pro Val Ser Gly Arg Arg Asp Thr Leu Ala Pro Asp Ser Leu Gln
        355                 360                 365
Ala Lys Glu Gly Asn Trp Val Pro Leu Ser Thr Trp Gly Thr Gly Gln
    370                 375                 380
Val Ala Pro Leu Thr Ala Val Pro Leu Ser Gly Gly Asp Gly Cys Ser
385                 390                 395                 400
Val Gly Met Pro Ser Lys Thr Glu Ala Val Val Ala Cys Ser Leu Cys
                405                 410                 415

(2) INFORMATION FOR SEQ ID NO: 9:
(i) SEQUENCE CHARACTERISTICS:
(A) LENGTH 1281 base pairs
(B) TYPE: nucleic acid
(C) STRANDEDNESS: single
(D) TOPOLOGY: linear
(ii) MOLECULE TYPE: cDNA to mRNA
(xi) SEQUENCE DESCRIPTION: SEQ ID NO: 9

ATGGTGGCCA GCGGTGGACG TCCTGATGGT CCCCCTTCTA TCACCCCCGA GAGCCCTCTC 60
ATTGTAGGTG GCAGAGAGTG GCAGGGTATG GCAGGTTCTT GCTGGAACAT CACCTACGTT 120
CAAGACTCGG TAGGACCTGC CACCAGCACC CTGATGTTTG TGGCTGGTGT GGTGGGCAAC 180
GGGCTAGCAC TGGGCATCCT GGGTGCCCGA CGGAGGTCAC ACCCATCGGC GTTTGCAGTG 240
TTGGTCACTG GGCTGGCAGT GACGGATCTG CTGGGCACGT GCTTCTTGAG CCCCGCGGTG 300
TTTGTGGCCT ATGCTCGAAA CAGCTCTTTG CTGGGCCTGG CCCACGGTGG GACGATGCTG 360
TGTGACACTT TCGCCTTCGC TATGACTTTC TTCGGCCTGG CCTCCACGCT CATCCTCTTC 420
GCCATGGCTG TGGAGCGGTG CCTGGCTCTC AGTCATCCCT ACCTGTACGC CCAGCTGGAC 480
GGGCCCCGCT GTGCCCGCTT GGCTTTGCCT GCCATCTACG CTTTCTGTTG TCTCTTCTGC 540
TCACTGCCCC TGCTGGGCCT GGGCGAGCAC CAGCAGTATT GTCCCGGGAG CTGGTGCTTC 600
ATCCGCATGC GTTCCCCCCA GCCTGGTGGC TGTGCCTTCT CCCTGGCCTA TGCCAGTCTC 660
ATGGCCCTGC TGGTGACCTC CATCTTCTTC TGCAACGGCT CCGTCACTCT CAGCCTCTGT 720
CACATGTACC GCCAACAGAG ACGGCACCAT GGCTCGTTTG TGCCGACCTC TCGGGCACGA 780
GAGGATGAAG TTTACCACCT GATTCTGCTG GCCCTCATGA CAGGCATCAT GGCCGTATGC 840
TCCCTGCCTC TCACGATCAG AGGATTCACG CAGGCCATCG CCCCAGACAG CAGAGAGATG 900
GGGGACCTCC ACGCCTTCCG TTTTAATGCC TTCAACCCCA TCCTGGACCC CTGGGTCTTC 960
ATCCTTTTCC GAAAGGCTGT CTTCCAACGC CTCAAGTTCT GGTTGTGTTG TCTGTGTGCC 1020
CGTTCTGTCC ATGGGGATCT GCAGACGCCC CTCTCCCGGC CCGTGTCGGG GAGAAGAGAC 1080
ACACTGGCTC CCGATTCTCT CCAGGCTAAG GAAGGGAACT GGGTGCCCCT GTCAACCTGG 1140
GGTACGGGGC AGGTGGCACC ATTGACTGCT GTGCCTCTGT CTGGTGGTGA TGGCTGTTCT 1200
GTGGGAATGC CATCCAAAAC AGAGGCTGTG GTTGCCTGCT CCCTCTGCTG ATACCTAAGC 1260
TGGTCCTGCT TGCCATCCCG G 1281

(2) INFORMATION FOR SEQ ID NO: 10:

(i) SEQUENCE CHARACTERISTICS:
(A) LENGTH 1387 base pairs
(B) TYPE: nucleic acid
(C) STRANDEDNESS: single
(D) TOPOLOGY: linear
(ii) MOLECULE TYPE: cDNA to mRNA
(xi) SEQUENCE DESCRIPTION: SEQ ID NO: 10

CGGTGAAACA GGAGAGGGAC AGACCTGGAG AAGACGGAAA CAAAAGACAG AAACACACGC 60
ACACGGGGAA CTGGGAGTTG ACTGGCAGAT CCCAGCTGCC CTGAGGATGG TGGCCAGCGG 120
TGGACGTCCT GATGGTCCCC CTTCTATCAC CCCCGAGAGC CCTCTCATTG TAGGTGGCAG 180
AGAGTGGCAG GGTATGGCAG GTTCTTGCTG GAACATCACC TACGTTCAAG ACTCGGTAGG 240
ACCTGCCACC AGCACCCTGA TGTTTGTGGC TGGTGTGGTG GGCAACGGGC TAGCACTGGG 300
CATCCTGGGT GCCCGACGGA GGTCACACCC ATCGGCGTTT GCAGTGTTGG TCACTGGGCT 360
GGCAGTGACG GATCTGCTGG GCACGTGCTT CTTGAGCCCC GCGGTGTTTG TGGCCTATGC 420
TCGAAACAGC TCTTTGCTGG GCCTGGCCCA CGGTGGGACG ATGCTGTGTG ACACTTTCGC 480
CTTCGCTATG ACTTTCTTCG GCCTGGCCTC CACGCTCATC CTCTTCGCCA TGGCTGTGGA 540
GCGGTGCCTG GCTCTCAGTC ATCCCTACCT GTACGCCCAG CTGGACGGGC CCCGCTGTGC 600
CCGCTTGGCT TTGCCTGCCA TCTACGCTTT CTGTTGTCTC TTCTGCTCAC TGCCCCTGCT 660
GGGCCTGGGC GAGCACCAGC AGTATTGTCC CGGGAGCTGG TGCTTCATCC GCATGCGTTC 720
CCCCCAGCCT GGTGGCTGTG CCTTCTCCCT GGCCTATGCC AGTCTCATGG CCCTGCTGGT 780
GACCTCCATC TTCTTCTGCA ACGGCTCCGT CACTCTCAGC CTCTGTCACA TGTACCGCCA 840
ACAGAGACGG CACCATGGCT CGTTTGTGCC GACCTCTCGG GCACGAGAGG ATGAAGTTTA 900
CCACCTGATT CTGCTGGCCC TCATGACAGG CATCATGGCC GTATGCTCCC TGCCTCTCAC 960
GATCAGAGGA TTCACGCAGG CCATCGCCCC AGACAGCAGA GAGATGGGGG ACCTCCACGC 1020
CTTCCGTTTT AATGCCTTCA ACCCCATCCT GGACCCCTGG GTCTTCATCC TTTTCCGAAA 1080
GGCTGTCTTC CAACGCCTCA AGTTCTGGTT GTGTTGTCTG TGTGCCCGTT CTGTCCATGG 1140
GGATCTGCAG ACGCCCCTCT CCCGGCCCGT GTCGGGGAGA AGAGACACAC TGGCTCCCGA 1200
TTCTCTCCAG GCTAAGGAAG GGAACTGGGT GCCCCTGTCA ACCTGGGGTA CGGGGCAGGT 1260
GGCACCATTG ACTGCTGTGC CTCTGTCTGG TGGTGATGGC TGTTCTGTGG GAATGCCATC 1320
CAAAACAGAG GCTGTGGTTG CCTGCTCCCT CTGCTGATAC CTAAGCTGGT CCTGCTTGCC 1380
ATCCCGG 1387

(2) INFORMATION FOR SEQ ID NO: 11:
(i) SEQUENCE CHARACTERISTICS:
(A) LENGTH 1320 base pairs
(B) TYPE: nucleic acid
(C) STRANDEDNESS: single
(D) TOPOLOGY: linear
(ii) MOLECULE TYPE: cDNA to mRNA

(vi) ORIGINAL SOURCE
(A) ORGANISM: rat
(F) TISSUE TYPE: lung
(ix) FEATURE
(A) NAME/KEY: CDS
(B) LOCATION: 107 to 1354
(C) IDENTIFICATION METHOD: P
(xi) SEQUENCE DESCRIPTION: SEQ ID NO: 11

CGGTGAAACA GGAGAGGGAC AGACCTGGAG AAGACGGAAA CAAAAGACAG AAACACACGC 60
ACACGGGGAA CTGGGAGTTG ACTGGCAGAT CCCAGCTGCC CTGAGG ATG GTG GCC 115
Met Val Ala
1
AGC GGT GGA CGT CCT GAT GGT CCC CCT TCT ATC ACC CCC GAG AGC CCT 163
Ser Gly Gly Arg Pro Asp Gly Pro Pro Ser Ile Thr Pro Glu Ser Pro
    5                   10                  15
CTC ATT GTA GGT GGC AGA GAG TGG CAG GGT ATG GCA GGT TCT TGC TGG 211
Leu Ile Val Gly Gly Arg Glu Trp Gln Gly Met Ala Gly Ser Cys Trp
20                  25                  30                  35
AAC ATC ACC TAC GTT CAA GAC TCG GTA GGA CCT GCC ACC AGC ACC CTG 259
Asn Ile Thr Tyr Val Gln Asp Ser Val Gly Pro Ala Thr Ser Thr Leu
                40                  45                  50
ATG TTT GTG GCT GGT GTG GTG GGC AAC GGG CTA GCA CTG GGC ATC CTG 307
Met Phe Val Ala Gly Val Val Gly Asn Gly Leu Ala Leu Gly Ile Leu
            55                  60                  65
GGT GCC CGA CGG AGG TCA CAC CCA TCG GCG TTT GCA GTG TTG GTC ACT 355
Gly Ala Arg Arg Arg Ser His Pro Ser Ala Phe Ala Val Leu Val Thr
        70                  75                  80
GGG CTG GCA GTG ACG GAT CTG CTG GGC ACG TGC TTC TTG AGC CCC GCG 403
Gly Leu Ala Val Thr Asp Leu Leu Gly Thr Cys Phe Leu Ser Pro Ala
    85                  90                  95
GTG TTT GTG GCC TAT GCT CGA AAC AGC TCT TTG CTG GGC CTG GCC CAC 451
Val Phe Val Ala Tyr Ala Arg Asn Ser Ser Leu Leu Gly Leu Ala His
100                 105                 110                 115
GGT GGG ACG ATG CTG TGT GAC ACT TTC GCC TTC GCT ATG ACT TTC TTC 499
Gly Gly Thr Met Leu Cys Asp Thr Phe Ala Phe Ala Met Thr Phe Phe
                120                 125                 130
GGC CTG GCC TCC ACG CTC ATC CTC TTC GCC ATG GCT GTG GAG CGG TGC 547
Gly Leu Ala Ser Thr Leu Ile Leu Phe Ala Met Ala Val Glu Arg Cys
            135                 140                 145
CTG GCT CTC AGT CAT CCC TAC CTG TAC GCC CAG CTG GAC GGG CCC CGC 595
Leu Ala Leu Ser His Pro Tyr Leu Tyr Ala Gln Leu Asp Gly Pro Arg
        150                 155                 160
TGT GCC CGC TTG GCT TTG CCT GCC ATC TAC GCT TTC TGT TGT CTC TTC 643

Cys Ala Arg Leu Ala Leu Pro Ala Ile Tyr Ala Phe Cys Cys Leu Phe
    165                 170                 175
TGC TCA CTG CCC CTG CTG GGC CTG GGC GAG CAC CAG CAG TAT TGT CCC 691
Cys Ser Leu Pro Leu Leu Gly Leu Gly Glu His Gln Gln Tyr Cys Pro
180                 185                 190                 195
GGG AGC TGG TGC TTC ATC CGC ATG CGT TCC CCC CAG CCT GGT GGC TGT 739
Gly Ser Trp Cys Phe Ile Arg Met Arg Ser Pro Gln Pro Gly Gly Cys
                200                 205                 210
GCC TTC TCC CTG GCC TAT GCC AGT CTC ATG GCC CTG CTG GTG ACC TCC 787
Ala Phe Ser Leu Ala Tyr Ala Ser Leu Met Ala Leu Leu Val Thr Ser
            215                 220                 225
ATC TTC TTC TGC AAC GGC TCC GTC ACT CTC AGC CTC TGT CAC ATG TAC 835
Ile Phe Phe Cys Asn Gly Ser Val Thr Leu Ser Leu Cys His Met Tyr
        230                 235                 240
CGC CAA CAG AGA CGG CAC CAT GGC TCG TTT GTG CCG ACC TCT CGG GCA 883
Arg Gln Gln Arg Arg His His Gly Ser Phe Val Pro Thr Ser Arg Ala
    245                 250                 255
CGA GAG GAT GAA GTT TAC CAC CTG ATT CTG CTG GCC CTC ATG ACA GGC 931
Arg Glu Asp Glu Val Tyr His Leu Ile Leu Leu Ala Leu Met Thr Gly
260                 265                 270                 275
ATC ATG GCC GTA TGC TCC CTG CCT CTC ACG ATC AGA GGA TTC ACG CAG 979
Ile Met Ala Val Cys Ser Leu Pro Leu Thr Ile Arg Gly Phe Thr Gln
                280                 285                 290
GCC ATC GCC CCA GAC AGC AGA GAG ATG GGG GAC CTC CAC GCC TTC CGT 1027
Ala Ile Ala Pro Asp Ser Arg Glu Met Gly Asp Leu His Ala Phe Arg
            295                 300                 305
TTT AAT GCC TTC AAC CCC ATC CTG GAC CCC TGG GTC TTC ATC CTT TTC 1075
Phe Asn Ala Phe Asn Pro Ile Leu Asp Pro Trp Val Phe Ile Leu Phe
        310                 315                 320
CGA AAG GCT GTC TTC CAA CGC CTC AAG TTC TGG TTG TGT TGT CTG TGT 1123
Arg Lys Ala Val Phe Gln Arg Leu Lys Phe Trp Leu Cys Cys Leu Cys
    325                 330                 335
GCC CGT TCT GTC CAT GGG GAT CTG CAG ACG CCC CTC TCC CGG CCC GTG 1171
Ala Arg Ser Val His Gly Asp Leu Gln Thr Pro Leu Ser Arg Pro Val
340                 345                 350                 355
TCG GGG AGA AGA GAC ACA CTG GCT CCC GAT TCT CTC CAG GCT AAG GAA 1219
Ser Gly Arg Arg Asp Thr Leu Ala Pro Asp Ser Leu Gln Ala Lys Glu
                360                 365                 370
GGG AAC TGG GTG CCC CTG TCA ACC TGG GGT ACG GGG CAG GTG GCA CCA 1267
Gly Asn Trp Val Pro Leu Ser Thr Trp Gly Thr Gly Gln Val Ala Pro
            375                 380                 385
TTG ACT GCT GTG CCT CTG TCT GGT GGT GAT GGC TGT TCT GTG GGA ATG 1315
Leu Thr Ala Val Pro Leu Ser Gly Gly Asp Gly Cys Ser Val Gly Met
        390                 395                 400
CCA TCC AAA ACA GAG GCT GTG GTT GCC TGC TCC CTC TGC TGATACCTAA 1364

Pro Ser Lys Thr Glu Ala Val Val Ala Cys Ser Leu Cys
    405                 410                 415
GCTGGTCCTG CTTGCCATCC CGG 1387

## Description

### Field of the Invention

This invention relates to a Prostaglandin I₂ (PGI₂) receptor. More particularly, it relates to a human or a rat PGI₂ receptor polypeptides, a process for the preparation thereof, DNAs encoding the said polypeptides, a replication or a expression vector comprising the said DNAs, a host cell transfected or transformed with the said vector, a monoclonal or polyclonal antibody against the said polypeptide, a pharmaceutical composition comprising the said polypeptides or antibody or a method of screening a compound possessing PGI₂, agonistic or antagonistic activity by using the said polypeptide or the said host cell.

### Background

Prostanoids such as Prostaglandin (PG), Thromboxane (TX) and Leukotriene (LT) are a family of oxidized metabolites of arachidonic acid and exhibit various physiological activities required to maintain local homeostasis in organisms (see The Pharmacological Basis of Therapeutics (Gilman, A. G., Good-man, L. S., Rail, T. W., and Murad, F., eds) 7th Ed., pp 660, Macmillan Publishing Co., New York (1985)).

These physiological activities are controlled by a cell membrane receptor specific for each prostanoid (see Annu. Rev. Pharm. Tox., 10, 213 (1989) and Prostanoids and their Receptors. In Comprehensive Medicinal Chemistry., pp 643 (1990), Pergamon Press, Oxford).

Prostacycline (PGI₂) is a very unstable compound which is one of prostanoids and it is largely produced by endothelial cells in blood vessels. PGI₂ inhibits blood platelet aggregation and induces vasodilatation by activating adenylate cyclase and generating cAMP. These actions can antagonize the strong blood platelet aggregation and vasoconstriction activities of TXA₂ which is another unstable prostanoid. PGI₂ contributes to the maintenance of homeostasis in blood circulation system (see Br. J. Pharmacol., 76, 3 (1982)). These activities of PGI₂ may be regarded as useful for prevention and/or treatment of diseases such as cerebral thrombosis and myocardial infarction. A lot of stable PGI₂ derivatives have been synthesized for drug development.

By using PGI₂ as well as these stable derivatives, the activities of PGI₂ have been studied extensively in blood platelets, smooth muscle, other tissues and various cells (see Prostanoids and their Receptors. In Comprehensive Medicinal Chemistry., pp 643 (1990), Pergamon Press, Oxford). These studies have revealed that responses to PGI₂ and its derivatives are somehow-different among the tissues and cells of different species.

This difference suggests the presence of receptor subtypes or coupling of the PGI₂ receptor to two or more G proteins. In addition, it is suggested that PGI₂ and its derivatives cause cross-reaction to the other subtype of prostanoid receptors (see Br. J. Pharmacol., 76, 149 (1982) and., 97, 657 (1989)). In order to clarify these points, it is regarded as necessary to analyze the structure, signalling and distribution of PGI₂ receptor.

### The disclosure of the invention

Recently, the present inventors et al. have succeeded in cloning a cDNA for a human and a mouse TXA₂ receptors and three subtypes of a mouse PGE receptor, i.e., EP₁, EP₂ and EP₃ (see Nature, 349, 617 (1991); Biochem. Biophys. Res. Commun., 184, 1197, (1992); J. Biol. Chem., 268, 20175 (1993); J. Biol. Chem., 268, 7759 (1993) and J. Biol. Chem., 267, 6463 (1992)). In addition, a cDNA for a mouse PGI₂ receptor has been cloned (presented at International Symposium on Molecular Biology of the Arachidonate Cascade in December 6-7, 1993).

By using a cDNA encoding a mouse PGI₂ receptor or its fragment as a probe, the present inventors et al. have carried out a homology search in human and rat cDNA libraries. From the results of sequencing isolated clones and expressing them, it has been shown that there are corresponding clones encoding a human and a rat PGI₂ receptors. Thus, the present invention has been derived.

Previously there was no polypeptide having an amino acid sequence identical to that of the polypeptide of the present invention, when the amino acid sequence of the polypeptide was compared by computer to all known sequences in data base of Swiss Prot (Swiss Prot Release 20). Furthermore, there was no nucleotide sequence which was identical to that encoding the polypeptide of the present invention, when the nucleotide sequence was compared by computer to all known sequences in data base of GenBank (GenBank Release 70.0). Therefore, it has been confirmed that the polypeptides of the present invention are entirely novel ones.

That is to say, the present invention relates to
1) a human PGI₂ receptor in substantially purified form,
2) a receptor described in the above 1), which is a polypeptide comprising an amino acid sequence shown in Seq. ID No. 1 or homologue thereof, or a fragment of the said sequence or homologue of the said fragment,
3) a polypeptide described in the above 2), consisting of the amino acid sequence shown in Seq. ID No. 1,
4) a DNA encoding the polypeptide described in the above 1), 2) or 3).
5) a DNA described in the above 4), having nucleotide sequence shown in Seq. ID No. 2 or a fragment capable of hybridizing selectively to the said sequence,
6) a DNA described in the above 4), having nucleotide sequence shown in Seq. ID No. 5 or a fragment capable of hybridizing selectively to the said sequence,
7) a DNA described in the above 4), having nucleotide sequence shown in Seq. ID No. 3 or a fragment capable of hybridizing selectively to the said sequence,
8) a DNA described in the above 4), having nucleotide sequence shown in Seq. ID No. 6 or a fragment capable of hybridizing selectively to the said sequence,
9) a rat PGI₂ receptor in substantially purified form,
10) a receptor described in the above 9), which is a polypeptide comprising an amino acid sequence shown in Seq. ID No. 8 or homologue thereof, or a fragment of the said sequence or homologue of the said fragment,
11 ) a polypeptide described in the above 1 0), consisting of the amino acid sequence shown in Seq. ID No. 8,
12) a DNA encoding the polypeptide described in the above 9), 10) or 11),
13) a DNA described in the above 12), having nucleotide sequence shown in Seq. ID No. 9 or a fragment capable of hybridizing selectively to the said sequence,
14) a DNA described in the above 12), having nucleotide sequence shown in Seq. ID No. 10 or a fragment capable of hybridizing selectively to the said sequence,
15) a replication or expression vector comprising DNA described in the above 4) to 8) or 12) to 14),
16) a host cell transformed or transfected with a replication or expression vector described in the above 15),
17) a process for the preparation of a polypeptide described in the above 1 ) to 3) or 9) to 11), which consists of culturing a host cell described in the above 16) under conditions effective to express the said polypeptide,
18) a monoclonal or polyclonal antibody against a polypeptide described in the above 1) to 3) or 9) to 11),
19) a pharmaceutical composition which is characterized by comprising a polypeptide described in the above 1) to 3) or 9) to 11), or an antibody described in the above 18) and a pharmaceutically acceptable excipient and/or carrier,
20) a method for screening a compound possessing PGI₂ agonistic or antagonistic activity which is characterized by using a polypeptide described in the above 1) to 3) or 9) to 11),
21) a method for. screening described in the above 18) which is characterized by using a host cell described in the above 16).

### Brief description of the drawing

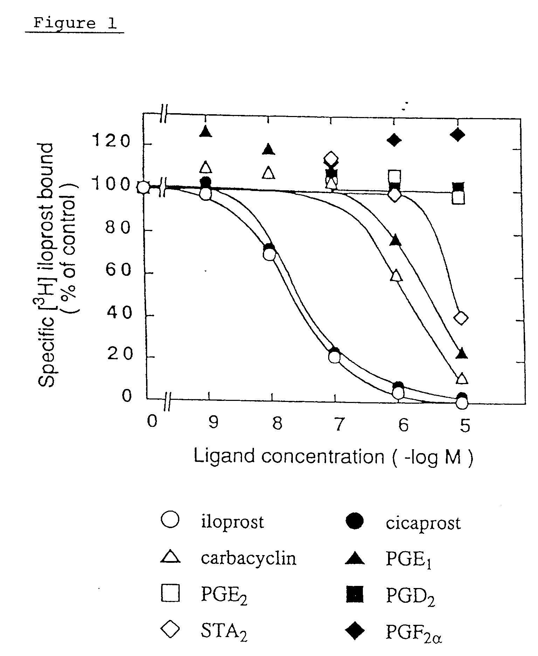

Figure 1 showed the inhibitory activities of a various ligands on binding [³H]iloprost onto the membrane of the cell transfected with MK71.

### Detailed description of the invention

The present invention relates to a human or a rat PGI₂ receptors in substantially purified form. Specifically, the human PGI₂ receptor of the present invention relates to a polypeptide comprising an amino acid sequence shown in Seq. ID No. 1 or homologue thereof, or a fragment of the said sequence or homologue of the said fragment. In addition, the present invention relates to DNAs encoding these polypeptides. More specifically, the present invention relates to DNAs having nucleotide sequence shown in Seq. ID No. 2, 3, 5 or 6 or DNAs containing a fragment capable of hybridizing selectively to the nucleotide sequence shown in Seq.ID No.2, 3, 5 or 6. The mouse PGI₂ receptor of the present invention relates to a polypeptide comprising an amino acid sequence shown in Seq. ID No. 8 or homologue thereof, or a fragment of the said sequence or homologue of the said fragment; and DNAs encoding these polypeptides, more specifically, DNAs having nucleotide sequence shown in Seq. ID No. 9 or 10 or a DNA containing a fragment shown in Seq. ID No. 9 or 10 or a DNA containing a fragment capable of hybridizing selectively to the nucleotide sequence shown in Seq. ID No. 9 or 10.

More particularly, the present invention relates to
(1) polypeptides comprising the amino acid sequences shown in Seq. ID No. 1 or 8,
(2) DNAs encoding the polypeptides described in the above (1),
(3) DNAs having nucleotide sequences shown in Seq. ID No. 2, 5 or 9 and
(4) DNAs having nucleotide sequences shown in Seq. ID No. 3, 6 or 10.

The polypeptides comprising the amino acid sequences shown in Seq. ID No. 1 or 8 mean not only those having the amino acid sequences shown in Seq. ID No. 1 or 8, but also those with addition of polypeptides comprising amino acid sequence 20% or less, more preferable 5% or less numbers of total amino acids of polypeptides shown in Seq. ID No. 1 or 8 at N- and/or C-terminal of the said polypeptides.

The polypeptides comprising the amino acid sequences shown in Seq. ID No. 1 or 8 in purified form mean generally the polypeptide in a preparation in which 90% or more, e.g. 95%, 98% or 99% of the polypeptide in the preparation is that of the Seq. ID No. 1 or 8.

Homologues of the polypeptides comprising the amino acid sequences shown in Seq. ID No. 1 or 8 will be generally at least 70%, preferably at least 80 or 90% and more preferably at least region of at least 100, preferably at least 150, for instance 200, 250 or 300 contiguous amino acids. Such homologues are to be included in the definition of the polypeptide of the present invention.

In addition, fragments of the polypeptides comprising the amino acid sequences shown in Seq. ID No. 1 or 8 or a fragment of homologues of the said polypeptide mean at least 10, preferably at least 15, for example 20, 25, 30, 40, 50 or 60 amino acids in length.

DNAs capable of hybridizing selectively to the DNAs having the nucleotide sequence shown in Seq. D No. 2 or 3, 5, 6, 9 or 10 will be generally at least 70%, preferably at least 80 or 90% and more preferably at least 95% homologous to the said DNA over a region of at least 100, preferably at least 1 50, for instance 200, 250 or 300 contiguous nucleotides. Such DNAs are to be included in the definition of the DNA of the present invention.

Fragments of the DNAs comprising the nucleotide sequence shown in Seq. ID No. 2, 3, 5, 6, 9 or 10 will be at least 10, preferably at least 15, for example 20, 25, 30 or 40 nucleotides in length, and are also included in the present invention.

It is possible to prepare a DNA of the present invention according to genetic engineering, chemical synthesis or methods known to the person skilled in the art.

A further embodiment of the invention provides replication and expression vectors comprising DNA of the present invention. The vectors may be, for example, plasmid, virus or phage vectors provided with an origin of replication, optionally a promoter for the expression of the said DNA and optionally a regulator of the promoter. The vector may contain one or more selectable marker promoter. The vector may contain one or more selectable marker genes, for example an ampicillin resistance gene.

A further embodiment of the invention provides host cells transformed or transfected with the vectors for the replication and expression of DNA of the present invention, including the DNA comprising the nucleotide sequence shown in Seq. ID No. 2, 3, 5, 6, 9 or 10 or the open reading frame thereof. The cells may for example be bacterial, yeast, insect cell or mammalian cell. A further embodiment of the invention provides a method of preparation of a polypeptide of the present invention which comprises culturing host cells of the present invention under conditions effective to express a polypeptide of the invention.

The polypeptide of the present invention includes one in which a part of their amino acid sequence is lacking (e.g., a polypeptide comprised of the only essential sequence for revealing a biological activity in mature protein), one in which a part of their amino acid sequence is replaced by other amino acids (e.g., those replaced by an amino acid having a similar property) and one in which other amino acids are added or inserted into a part of their amino acid sequence, as well as ones having the amino acid sequence shown in Seq. ID No. 1 or 8.

It is well known that there can be between one and six kinds of codon encoding for one amino acid (for example, one kind of codon for methionine (Met), and six kinds of codon for leucine (Leu) are known). Accordingly, the nucleotide sequence of DNA can be changed in order to encode the polypeptide having the same amino acid sequence.

The DNAs of the present invention, specified in (2) include a group of every nucleotide sequences encoding polypeptides shown yield of production of a polypeptide by changing a nucleotide sequence.

The DNAs specified in (3) are the embodiment of DNA shown in (2), and are the natural form of the sequence.

The DNAs shown in (4) indicate the sequence of the DNAs specified in (3) with a untranslated region.

The DNAs having a nucleotide sequence shown in Seq. ID No. 3, 6 or 10 may be prepared according to the following methods, that is:
i) by isolating mRNA from a cell which produces the polypeptide of the present invention,
(ii) by preparing a first strand (single strand cDNA) from mRNA thus obtained, followed by preparing a second strand (double strand cDNA) (synthesis of cDNA),
iii) by inserting cDNA thus obtained into a proper plasmid vector,
iv) by transfecting recombinant vector into host cells (construction of cDNA library),
v) by isolating a plasmid comprising the desired DNA with plaque hybridization from a cDNA library thus obtained,
vi) by determining nucleotide sequence of the desired DNA.

Explained in detail, step (i) may be carried out in accordance with the method of Okayama, H et al (described in Method in Enzymology, 154, 3, (1987)) or the method of Chirgwin, J. M. et al (described in Biochem., 18, 5294 (1979)) from the tissues which PGI₂ receptors may be expressed in human or rat, preferably cells of tissue or cells of the lung, blood vessel, leukocyte and blood platelet etc.

Step (ii), (iii) and (iv) are a series of steps for preparing a cDNA library, and may be carried out in accordance with the method of Glubler & Hoffman (described in Gene, 25, 263, (1983)) with a slight modification.

As examples of the plasmid vector used in the step (iii), many that function in an *E.coli* strain (e.g. pB322) and in Bacillus subtilis (e.g. pUB110) are known, and λ-ZAPII functioning in an *E.coli* strain is preferably used.

As for host cell used in step (iv), many are known. Any host cells can be used and DH5 competent cells (prepared according to the method described in Gene, 96, 23 (1990)) can be preferably used.

Step (v) itself is a known method, for example, this step may be carried out by the plaque hybridization method or the colony hybridization method (described in Gene, 10, 63 (1980)) etc. As for an adequate probe, a DNA for a PGI₂ receptor of another species of animal, a fragment thereof or a DNA having the homology to the said DNA can be used.

Step (vi) itself is known method, for example this step may be carried out by the dideoxy terminator method or Maxam-Gilbert method.

Once the nucleotide sequences shown in Seq. ID No. 2, 3, 5, 6, 9 or 10 are determined, DNA of the present invention may be obtained by chemical synthesis, by PCR (polymerase chain reaction) method or by hybridization making use of a fragment of the said DNA of the present invention, as a probe. Furthermore, DNA of the present invention may be obtained in a desired amount by transforming with a vector DNA containing a DNA of the present invention into a proper host, followed by culturing the transformant.

The polypeptides of the present invention (Seq. ID No. 1 or 8) may be prepared by:
1) isolating and purifying from an organism or a cultured cell,
2) chemically synthesizing, or
3) using a skill of genetic engineering etc., preferably, by the method described in (3).

Examples of expression systems (host cell-vector system) to prepare a polypeptide by using genetic engineering, are, for example, the expression system of bacteria, yeast, insect cells and mammalian cells.

For example, expression in *E.coli* may be carried out by connecting a DNA encoding the mature protein (e.g., DNA encoding nucleotide sequence shown in Seq. ID No. 2, 5 or 9) thus obtained to the downstream of an appropriate promoter (e.g., trp promoter, lac promoter, λPL promoter, T7 promoter etc.), and then inserting it into a vector (e.g., pBR322, pUC18, pUC19 etc.) which functions in an *E.coli* strain to prepare an expression vector. And then, by culturing the (e.g., *E.coli* DH1, JM109, *E.coli* HB101) transfected with the expression vector thus obtained in an appropriate medium, the desired polypeptides may be prepared from the obtained bacteria. When a bacterial signal peptide (e.g., signal peptide of pel B) is utilized, the desired polypeptide may be also produced in the periplasm. Furthermore, a fusion protein with another polypeptide may be also produced easily.

Furthermore, the expression in a mammalian cell may be carried out, for example, by inserting the DNA shown in Seq. ID No. 3, 6 or 10 downstream of an appropriate promoter (e.g., SV40 promoter, LTR promoter, metallothionein promoter etc.) in an appropriate vector (e.g., retrovirus vector, papilloma virus vector, vaccinia virus vector, SV40 vector) to obtain an expression vector, and transfecting an appropriate mammalian cell (e.g., monkey COS-7 cell, Chinese hamster CHO cell, mouse L cell) with the expression vector thus obtained, and then culturing the transformant in an appropriate medium to get a desired polypeptide in the culture medium. The polypeptide thus obtained may be isolated and purified by conventional biochemical methods.

For the treatment etc. of the hemorrhagic disease, the polypeptides of the present invention may be normally administered systematically or partially, usually by oral or parenteral administration, preferably orally, intravenously or intraventricularly.

The doses to be administered are determined depending upon age, body weight, symptom, the desired therapeutic effect, the route of administration, and the duration of the treatment. In the human adult, the doses per person per dose are generally between 100 µg and 100 mg, by oral administration, up to several times per day, and between 10 µg and 100 mg, by parenteral administration up to several times per day.

As mentioned above, the doses to be used depend upon various conditions. Therefore, there are cases in which doses lower than or greater than the ranges specified above may be used.

On administration of the compounds of the present invention, it may be used as solid compositions, liquid compositions or other compositions for oral administration, as injections, liniments or suppositories for parenteral administration.

Solid compositions for oral administration include compressed tablets, pills, capsules, dispersible powders, granules. Capsules include soft capsules and hard capsules.

In such compositions, one or more of the active compound(s) is or are admixed with at least one inert diluent (such as lactose, mannitol, glucose, hydroxypropyl cellulose, microcrystalline cellulose, starch, polyvinylpyrrolidone, magnesium metasilicate aluminate). The compositions may also comprise, as is normal practice, additional substances other than inert diluents: e.g. lubricating agents (such as magnesium stearate etc.), disintegrating agents (such as cellulose calcium glycolate), stabilizing agents (such as human serum albumin, lactose etc.), and assisting agents for dissolving (such as arginine, asparaginic acid).

The tablets or pills may, if desired, be coated with a film of gastric or enteric material (such as sugar, gelatin, hydroxypropyl cellulose or hydroxypropylmethyl cellulose phthalate), or be coated with two or more films. And further, coating may include containment within capsules of absorbable materials such as gelatin.

Liquid compositions for oral administration include pharmaceutically-acceptable emulsions, solutions, suspensions, syrups and elixirs etc. and may also contain inert diluent(s) commonly used in the art (for example, purified water, ethanol). Besides inert diluents, such compositions may also comprise adjuvants such as wetting agents, suspending agents, sweetening agents, flavouring agents, perfuming agents and preserving agents.

Other compositions for oral administration include spray compositions which may be prepared by known methods and which comprise one or more of the active compound(s). Spray compositions may comprise additional substances other than inert diluents: e.g. stabilizing agents such as sodium hydrogen sulfate, stabilizing agents to give the title compound isotonicity, isotonic buffer such as sodium chloride, sodium citrate, citric acid. For preparation of such spray compositions, for example, the method described in the United States Patent No. 2868691 or 3095355 may be used.

Injections for parenteral administration include sterile aqueous or non-aqueous solutions, suspensions and emulsions. As for aqueous solutions or non-aqueous solutions or suspensions, one or more active compound(s) is (are) admixed with at least one inert diluent. Aqueous dilution include distilled water for injection and physiological salt solution. Non-aqueous dilution include propylene glycol, polyethylene glycol, plant oil such as olive oil, alcohol such as ethanol, POLYSOLBATE80 (registered trade mark) etc.

Injections may comprise additional other than inert diluents: e.g. preserving agents, wetting agents, emulsifying agents, dispersing agents, stabilizing agent (such as human serum albumin, lactose), and assisting agents such as assisting agents for dissolving (arginine, asparaginic acid).

They may be sterilized for example, by filtration through a bacteria-retaining filter, by incorporation of sterilizing agents in the compositions or by irradiation. They may also be manufactured in the form of sterile solid compositions, (for example, by freeze-drying), and which can be dissolved in sterile water or some other sterile diluents for injection immediately before use.

Other compositions for parenteral administration include liquids for external use, and endermic liniments (such as ointments), suppositories for rectal administration and pessaries which comprise one or more of the active compound(s) and may be prepared by known methods.

### Best Mode to practice the invention

The following examples are illustrated by, but not limited to, the present invention.

### Reference Example 1

### Cloning of a cDNA for a mouse PGI₂ receptor

The total RNA of mouse P-815 cultured mastocytoma cell was isolated by acid guanidium thiocyanate-phenol-chloroform method (Anal. Biochem., 162, 156 (1987)). A single strand cDNA was synthesized using random primer (marketed from Toyobo Co., Ltd.) and Molony Murine Leukemia Virus reverse transcriptase (marketed from Bethesda Research Laboratories). As for PCR primers, Regions of the cDNA for a human and a mouse TXA₂ receptors and cDNA for three kinds of subtype of a mouse PGE receptor corresponding to parts encoding the second extracellular loop domain and the seventh transmembrane domain were used. That is to say, as for PCR primers, the synthesized nucleotides (a) and (b) as shown as the following formula encoding amino acid sequences of Leu-Ala-Val-Thr-Asp-Leu and Asp-Pro-Trp-lie-Tyr-Ile, respectively, were used.
5'-CTG/CGCA/TGTG/CACNGAT/CT/CT-3' (a) and
5'-AT/GA/GTANACCCANGGA/GTC-3' (b)

With the single strand cDNA obtained above as a template, amplification was performed with 2 cycles of 1 minute denaturation at 94°C, 0.5 minute annealing at 40°C and 1 minute extension at 72°C; 3 cycles of 1 minute denaturation at 94°C, 0.5 minute annealing at 46°C and 1 minute extension at 72°C followed by 27 cycles of 1 minute denaturation at 94°C, 0.5 minute annealing at 5°C and 1 minute extension at 72°C. The amplified cDNA was blunted with DNA polymerase I, isolated by agarose gel electrophoresis and subcloned into the EcoRV site of pbluescript SK (+) Vector (marketed from Stratagene). From the result of sequencing a clone picked at random, the clone having 706 bp (named as CY) showed the relative homology to the nucleotide sequences of cDNA for the known prostanoid receptors, so such a clone was used as a probe for hybridization.

Polyadenylated RNA was isolated from P-815 cells. A cDNA was prepared by an oligo(dT) priming method using a cDNA synthesis kit (marketed from Pharmacia). After size-selection (more than 1.5 kb), the said cDNA was inserted into the EcoRI site of λ- ZAPIIDNA (marketed from Stratagene) using EcoRI adapters (marketed from New England Biolabs) to prepare the cDNA library. 3.5 x 10⁵ of clones in the obtained cDNA library were transferred to nylon membranes (Trade mark: Hybond-N plus, marketed from Amersham) and screened by cross-hybridization with using cDNA insert of the clone CY. Hybridization was carried out at 65°C in 6 x SSC (900 mM NaCl, 90 mM sodium citrate) containing 5 x Denhardt's solution (0.1 % Ficoll, 0.1% polyvinylpyrrolidone and 0.1% bovine serum albumin), 0.5% SDS (sodium dodecyl sulfate), 200 µg/ml heat-denatured salmon sperm DNA and the ³²P-radiolabeled cDNA insert of the clone CY for 15 hours. After then, filters were washed twice at 65°C in 2 x SSC containing 1% SDS for 30 minutes. Nine positive clones were picked and converted to plasmids by the in vivo excision method. Nucleotide sequences were determined on double strands with Bca Best DNA sequencing kit (marketed from Takara-Shuzo Co., Ltd.). A representative clone of CP302 was obtained.

### Example 1

### Cloning of a cDNA for a human PGI₂ receptor (derived from human blood platelet)

A cDNA was prepared by an oligo(dT) priming method from a human megakaryocyte leukemia cell (CMK cell, prepared by the method described in Brit. J. Haematol., 72, 184 (1989)). After size-selection (more than 1.5 kb), the said cDNA was inserted into the EcoRI site of λ- ZAPIIDNA (marketed from Stratagene) using EcoRI adapters (marketed from New England Biolabs) to prepare the cDNA library. 3.5 x 10⁵of clones in the obtained cDNA library were transferred to nylon membranes (Trade mark: Hybond-N plus, marketed from Amersham) and screened by cross-hybridization with using cDNA insert of the CY clone prepared in Reference Example 1. Hybridization was carried out at 65°C in 6 x SSC (900 mM NaCl, 90 mM sodium citrate) containing 5 x Denhardt's solution, 0.5% SDS, 200 µg/ml heat-denatured salmon sperm DNA and the ³²P-radiolabeled cDNA insert of the clone CY for 15 hours. After hybridization, filters were washed twice at 65°C in 2 x SSC containing 1% SDS for 30 minutes. Several positive clones were picked and subjected to sequence analysis.

Nucleotide sequences were determined on double strands by dideoxy chain termination method. From the result of the analysis, one representative clone, MK71, was full length cDNA having open reading frame (shown in Seq. ID No. 2) of 1158 bp. Full length nucleotide sequence was shown in Seq. ID No. 3. Predicted amino acid sequence from the open reading frame was shown in Seq. ID No. 1. Nucleotide sequence of MK71 as shown in Seq. ID No. 3 and the predicted amino acid sequence were shown together in Seq. ID No. 4.

### Example 2

### A cloning of cDNA-for human PGI₂ receptor (derived from human lung)

By using a cDNA library (Clontech Code No.: CHAL 1033a) derived from human lung and the fragment (about 1.5 kb) prepared by digesting CP302 prepared in Reference Example 1, with EcoRV, cross-hybridization was carried out according to the procedure as Example 1 under the following condition. Hybridization was carried out at 37°C in 5 x SSPE (0.75 M NaCl, 0.05 M NaH₂PO₄ - H₂0, 0.005 M EDTA.) containing 25% for amide, 1 x Denhardt's solution, 0.1% SDS, 100 µg/ml heat-denatured salmon sperm DNA and a probe radiolabeled with ³²P (the fragment prepared by digesting CP302 prepared in Reference Example 1, with EcoRV) for 20 hours. After hybridization, filters were washed twice at room temperature in 2 x SSC for 10 minutes, twice at 37°C in 2 x SSC for 10 minutes and one time at 42°C in 2 x SSC for 10 minutes, succeedingly. A lot of positive clones were picked up, subcloned into pBluescript KS (-) (marketed from Invitrogen Co., Ltd.) and subjected to sequence analysis.

Nucleotide sequences were determined on double strands by dideoxy chain termination method. From the result of the analysis, one representative clone, phIPR1, was full length cDNA clone having open reading frame (shown in Seq. ID No. 5) of 1158 bp. Full length nucleotide sequence was shown in Seq. ID No. 6. Predicted amino acid sequence from the open reading frame was shown in Seq. ID No. 1. Nucleotide sequence of phIPRl as shown in Seq. ID No. 6 and the predicted amino acid sequence were shown together in Seq. ID No. 7.

To compare with the nucleotide sequence shown in Seq. ID No. 2 and 5, the base at the 159th position in the nucleotide sequence of Seq. ID No. 2 was guanine "G", on the other hand, the corresponding of Seq. ID No. 5 was cytosine "C". In addition, the base at the 984th position in the nucleotide sequence of Seq. ID No. 2 was adenine "A", and the corresponding of Seq. ID No. 5 was cytosine "C". The similar difference exists in Seq. ID No. 3 and 6, and Seq. ID No. 4 and 7. It is thought that such differences may be result from the difference of cells used to prepare for cDNA library.

### Example 3

### Cloning a cDNA for a rat PGI₂ receptor (derived from rat lung)

By using a cDNA library derived from rat lung (Clontech Code No. CLRL 1008a) and fragment (1.5 kb) prepared by digesting CP302 prepared in Reference Example 1, with EcoRV, cross-hybridization was carried out according to the procedure as Example 2. Several positive clones were picked, subcloned into pBluescript KS (-) (marketed from Invitrogen Co., Ltd.) and subjected to sequence analysis.

Nucleotide sequences were determined on double strands by dideoxy chain termination method. From the result of the analysis, one representative clone, prIPR1, was full length cDNA clone having open reading frame (shown in Seq. ID No. 9) of 1248 bp. Full length nucleotide sequence was shown in Seq. ID No. 10. Predicted amino acid sequence from the open reading frame was shown in Seq. ID No. 8. Nucleotide sequence of prIPR1 as shown in Seq. ID No. 10 and the predicted amino acid sequence were shown together in Seq. ID No. 11.

### Example 4

### Expression in CHO cells and ligand binding assay

The EcoRI insert of cDNA of MK71 was subcloned into pdKCR-dhfr which was the expression vector for eukaryotic cell containing mouse gene as selection marker. The resultant plasmid DNA was transfected to dihydrofolate reductase-defective CHO cells (CHO-dhfr⁻) by lipofection method (described in Proc. Natl. Acad. Sci. USA, 84, 7413 (1987)). A group of cells expressing dihydrofolate reductase was cultured in the α-modified eagle medium (marketed from Cell Culture Laboratories) containing penicillin (100 units/ml), streptomycin (100 mg/ml) and 10% dialyzed fetal bovine serum and selected. From these groups of cells, the cell line was isolated by single cell cloning. A cDNA expression was assayed by RNA blotting analysis. The cell line prepared by the transfection to CHO cell with pdKCR-dhfr as control, showed no sign in RNA blotting analysis.

The transfected CHO cell clones were cultured, harvested and homogenized using a Potter-Eivehjem homogenizer in a solution consisting of 25 mM Tris-HCl (pH 7.5), 0.25 M sucrose, 10 mM MgCl₂, 1 mM EDTA and 0.1 mM phenylmethylsulfonylfluoride. The homogenate was centrifuged at 800 x g for 10 minutes. The supernatant was saved and the pellet was suspended in the solution consisting of 25 mM Tris-HCl (pH 7.5), 0.25 M sucrose, 10 mM MgCl₂, 1 mM EDTA and 0.1 mM phenylmethylsulfonylfluoride, homogenized and centrifuged again. The supernatant and the saved supernatant were combined and centrifuged at 100,000 x g for 1 hour. The pellet was suspended in 20 mM MES (2-(N-morpholino)ethane sulphonic acid-hydrate, pH 6.0), 10 mM MgCl₂ and 1 mM EDTA (the suspension buffer) and used asthe crude membrane tissue.

[³H]iloprost binding assay was carried out as follows. That is to say, the crude membrane tissue, 40 µg protein and [³H]iloprost with various concentrations (for Scatchard analysis) or 20 nM [³H]iloprost (for displacement experiment) were suspended in the suspension buffer in total volume of 100 ml and incubated at 30°C. for 1 hour. The incubation was terminated by the addition of 2 ml of the ice-chilled suspension buffer. The mixture was rapidly filtered through a Whatman GF/C filter. The filter was washed four times with 2 ml of the ice-chilled suspension buffer. The radioactivity on the filter was measured by the conventional methods. The result was shown in Figure 1.

This assay showed that [³H]iloprost which was the specific ligand on PGI₂ receptor, binded specifically onto the cell membrane of CHO cell expressing MK-71. Scatchard analysis of this binding showed a dissociation constant of 3.3 nM and maximal binding of 3.2 pmol/mg protein. In addition, inhibitory activity of various prostanoids on specific [³H]iloprost binding was shown in Figure 1. This showed the inhibitory activity of various prostanoids in the rank order of iloprost = cicaprost >> carbacyclin (all are stable PGI₂ agonists) > PGE1 > STA₂ (stable TXA₂ agonist) >> PGE₂ =PGD₂ =PGF₂a -

### Effect of the invention

The polypeptides themselves of the present invention can bind to PGI₂ specifically, so they may be used as agents for preventing or treating the diseases caused by excessive production of PGI₂, for example, hemorrhagic disease. They may be also used in screening a compound which can agonize or antagonize for PGI₂.

DNA of the present invention may also be inserted into the vectors described above in an antisense orientation in order to provide for the production of antisense RNA. Antisense RNA may also be produced by synthetic means. Such antisense RNA may be used in a method of controlling the levels of a polypeptide of the invention in a cell.

The present invention also provides pharmaceutical compositions comprising a polypeptide of the invention, or an antibody thereof, in association with a pharmaceutically acceptable excipient and/or carrier.

Further, polyclonal or monoclonal antibodies against the polypeptide of the present invention can be used in the determination of the amount of the said polypeptide in organism, and thereby, may be utilized for the purpose of investigating the relationship between the said polypeptide and diseases, or for the purpose of diagnosing diseases, and the like. Polyclonal and monoclonal antibody thereof may be prepared by conventional methods by using the said polypeptide or the fragment thereof as an antigen.

The DNA of the present invention may be utilized as an important and essential template in preparing the polypeptide of the present invention which is expected to possess various uses for diagnosis of and in the treatment of gene diseases. Further, genomic DNA may be isolated by using the DNA of the present invention as a probe. Similarly, it, is possible to isolate genes having high homology to the DNA of the present invention in human or those of other species.

## Claims

1. A human PGI₂ receptor in substantially purified form.

2. A receptor according to claim 1, which is a polypeptide comprising an amino acid sequence shown in Seq. ID No. 1 or homologue thereof, or a fragment of the said sequence or homologue of the said fragment.

3. A polypeptide according to claim 2, consisting of the amino acid sequence shown in Seq. ID No. 1.

4. A DNA encoding the polypeptide according to claim 1, 2 or 3.

5. A DNA according to claim 4, having nucleotide sequence shown in Seq. ID No. 2 or a fragment capable of hybridizing selectively to the said sequence.

6. A DNA according to claim 4, having nucleotide sequence shown in Seq. ID No. 5 or a fragment capable of hybridizing selectively to the said sequence.

7. A DNA according to claim 4, having nucleotide sequence shown in Seq. ID No. 3 or a fragment capable of hybridizing selectively to the said sequence.

8. A DNA according to claim 4, having nucleotide sequence shown in Seq. ID No. 6 or a fragment capable of hybridizing selectively to the said sequence.

9. A rat PGI₂ receptor in substantially purified form.

10. A receptor according to claim 9, which is a polypeptide comprising an amino acid sequence shown in Seq. ID No. 8 or homologue thereof, or a fragment of the said sequence or homologue of the said fragment.

11. A polypeptide according to claim 10, consisting of the amino acid sequence shown in Seq. ID No. 8.

12. A DNA encoding the polypeptide described in claim 9, 10 or 11.

13. A DNA according to claim 12, having nucleotide sequence shown in Seq. ID No. 9 or a fragment capable of hybridizing selectively to the said sequence.

14. A DNA according to claim 12, having nucleotide sequence shown in Seq. ID No. 10 or a fragment capable of hybridizing selectively to the said sequence.

15. A replication or expression vector comprising DNA described in claim 4 to 8 or 12 to 14.

16. A host cell transformed or transfected with a replication or expression vector described in claim 15.

17. A process for the preparation of a polypeptide described in claim 1 to 3 or 9 to 11, which is characterised by culturing a host cell described in claim 16 under the condition effective to express the said polypeptide.

18. A monoclonal or polyclonal antibody against a polypeptide described in claim 1 to 3 or 9 to 11.

19. A pharmaceutical composition which is characterized by comprising a polypeptide described in claim 1 to 3 or 9 to 11, or an antibody described in claim 18 and a pharmaceutically acceptable excipient and/or carrier.

20. A method for screening a compound possessing PGI₂ agonistic or antagonistic activity which is characterized by using a polypeptide described in claim 1 to 3 or 9 to 11.

21. A method for screening according to claim 18 which is characterized by using a host cell described in claim 16.
